# EUROPEAN PATENT APPLICATION

(11) **EP 4 850 696 A1**
(43) Date of publication of application: **09.12.2026**
(21) Application number: 25382583.0
(22) Date of filing: 06.06.2025
(51) Int. Cl.: A61K 47/54, A61K 47/69, A61P 29/00, A61P 11/00, A61P 19/00, A61P 35/00

(54) **THERAPEUTIC NANOVEHICLES FORMED BY MAGNETIC NANOPARTICLES AND LONG NON-CODING RNAS**

(71) Applicant: Universidad del País Vasco/Euskal Herriko Unibertsitatea, 48940 Leoia, Bikaia (ES)
(72) Inventor: RUBIO CASTELLANOS, Ainara, E-48940 Leioa - Vizcaya (ES); RUBIO CASTELLANOS, Idoia, E-48940 Leioa - Vizcaya (ES); ROJAS MÁRQUEZ, Henar, E-48940 Leioa - Vizcaya (ES); BARON TORRE, Ander, E-48940 Leioa - Vizcaya (ES); INSAUSTI PEÑA, Maite, E-48940 Leioa - Vizcaya (ES)
(74) Representative: ABG Intellectual Property Law, S.L.

(57) **Abstract**

The present invention relates to nanoparticles functionalized with long noncoding RNA and uses thereof, in particular in the treatment of diseases wherein the long noncoding RNAs have reduced expression, for example in the treatment of cancer.

## Description

### FIELD OF INVENTION

The field of invention is comprised within the field of biomedicine and nanobiotechnology. It relates to nanoparticles functionalized with long noncoding RNA and uses in medicine.

### BACKGROUND ART

Magnetic nanoparticles (MNPs) are highly valuable nano-devices in theranostic medicine because of their ability to integrate diagnosis, therapy, targeting capability and real-time monitoring in the same platform. Among all the iron oxide phases, magnetite (Fe₃O₄) is the preferred one for biomedical applications because of its low toxicity, good biodegradability, high saturation magnetization and multipurpose potential in the healthcare industry. Obviously, the success of any technological application relies on the quality of the material used. However, the case of iron oxide nanoparticles (IONPs) is rather complicated as even the most refined synthesis routes usually produce a mixture of non-stoichiometric magnetite, maghemite (γ-Fe₂O₃) and/or wüstite (FeO) phases, which leads to deteriorated magnetic properties. Consequently, there is important active worldwide research studying how certain synthetic parameters affect the crystallinity, stoichiometry, size/shape and anisotropy of magnetite NPs.

On the other hand, it is well reported so far that the biomagnetic performance of IONPs (such as the heating power, the magnetic-targeting or the contrast enhancement in MRI) in cellular environments decreases significantly and turns out to be quite unpredictable. The root of this problem lies in the large and chaotic nanoparticle agglomerations that take place when the colloid of MNPs is dispersed in saline surroundings with high viscosity. Therefore, one of the key points for a relevant improvement in this field is to attain MNP systems whose therapeutic/diagnostic efficiency stays unchangeable regardless of the ionic strength and viscosity of the biological media. Existing optimized protocols, allow coating the MNPs with amphiphilic copolymers (PMAO-PEG type) that give rise to magnetic nanoformulations with minimal aggregation degree, great magnetothermal action and maximal reproducibility. The consistency and predictability of these nanosystems can be considered a great step forward in the development of the emerging era of theranostics and the next generation of medical technologies.

In 2012, under the umbrella of the ENCODE project, several research groups claimed that 80% of the human genome, much of which had been previously considered junk, exhibited biochemical activities such as transcription and transcription factor binding. Among these previously neglected genomic regions, many are producing long noncoding RNAs (IncRNAs). IncRNAs are not translated into functional proteins and some of them have been described to regulate expression of other coding genes. The expression of IncRNAs is typically cell-lineage specific and they can function by interacting with other RNA, DNA or protein molecules, affecting several layers of genetic regulation, such as chromatin modification, mRNA biogenesis or protein synthesis. Although their functional characterization is a complicated task, the study of the molecular mechanism in which they are involved and their implication in the development of a wide range of pathologies is currently being studied. Significantly, alterations in the structure and function of specific IncRNAs have been related to different inflammatory mediated disorders, including intestinal related pathologies, cardiovascular diseases, neurodegenerative disorders or cancer.

In recent years, targeting IncRNAs for the development of therapies, mainly focused on different types of cancer, has drawn widespread attention. Some IncRNA based clinical trials have been already approved by the FDA for the treatment of different diseases. For instance, a short single stranded antisense oligonucleotide (ASO), called Andes-1537, targeting antisense non-coding mitochondrial RNAs, has been analyzed in a phase I clinical trial in patients with solid tumors and advanced metastatic cancer, being well tolerated by the recipients.

Despite this, there have been no advancements on the development of directed treatments using IncRNAs, therefore limiting the use of IncRNAs for treatment.

### SUMMARY OF INVENTION

The present invention provides an innovative non-viral RNA delivery system composed of nanoparticles with a magnetic core and an amphiphilic polymer outer shell, wherein the nanoparticles comprise long non-coding RNA (IncRNA), which produce an efficient nanovehicle capable of delivering specific information within the cell. One major advantage of our creation is that it is highly versatile; that is to say, the inorganic core and the type of IncRNA can be adapted to specific healthcare demands.

A first aspect of the present invention relates to a nanoparticle comprising a magnetic core and an outer shell, wherein the outer shell comprises a long noncoding RNA.

Another aspect of the present invention relates to a method for obtaining a nanoparticle according to the invention comprising the steps of:
a) providing a nanoparticle comprising a magnetic core and an outer shell wherein the outer shell is functionalized with a first member of a binding pair of molecules;
b) providing a long noncoding RNA functionalized with a second member of the binding pair of molecules;
c) bringing the nanoparticle of step a) into contact with the long noncoding RNA of step b) in conditions such that the second member of the binding pair of molecules binds to the first member of a binding pair of molecules.

A further aspect of the present invention relates to a method for obtaining a nanoparticle according to the invention, wherein the magnetic core of the nanoparticle is obtained by a method comprising the steps of:
a) providing a suspension comprising the Fe₃O₄ magnetic core;
b) providing a solution comprising the PMAO-PEG copolymer;
c) mixing the suspension of step a) and the solution of step b) such that the PMAO-PEG copolymer binds to and coats the Fe₃O₄ magnetic core, obtaining nanoparticles;
d) functionalizing the PMAO-PEG copolymer of the nanoparticles obtained in step c) with avidin protein;
e) bringing into contact the nanoparticles obtained in step d) with a long noncoding RNA functionalized with biotin, such that the biotin binds to the avidin protein.

Yet another aspect of the present invention relates to the nanoparticles according to the invention for use in medicine.

Another aspect of the present invention relates to the nanoparticles according to the invention for use in the treatment of a disease which is treatable by expression of the long noncoding RNA present in the nanoparticles.

One more aspect of the present invention relates to the nanoparticles according to the invention for use in the treatment of chronic inflammation, chronic wounds and/or cancer.

A further aspect of the present invention relates to a method to increase endogenous levels of a long noncoding RNA comprising the step of transfecting cells with the nanoparticles according to the invention.

### BRIEF DESCRIPTION OF THE FIGURES

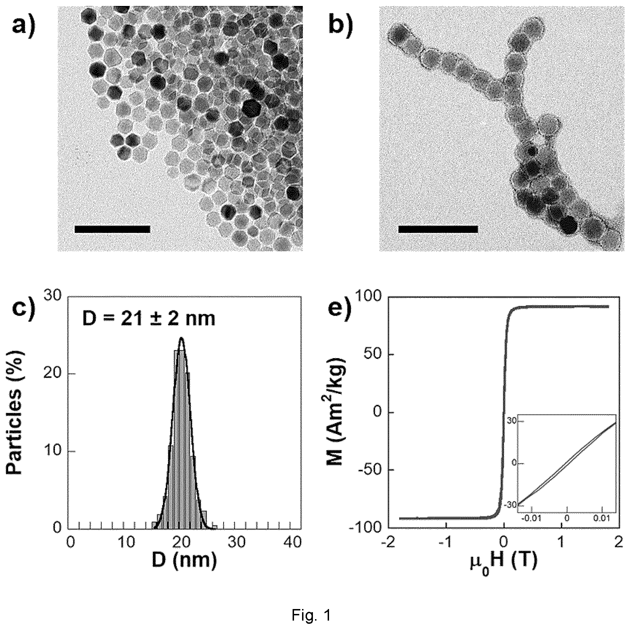

**Figure 1****.** a) TEM micrograph of the initially synthesized Fe₃O₄ NPs, b) TEM micrograph of the Fe₃O₄ NPs surrounded by PMAO-PEG copolymer (scale bars 100 nm), c) size distribution of the initially synthesized NPs and d) M(H) curve at RT measured in a DC SQUID magnetometer.

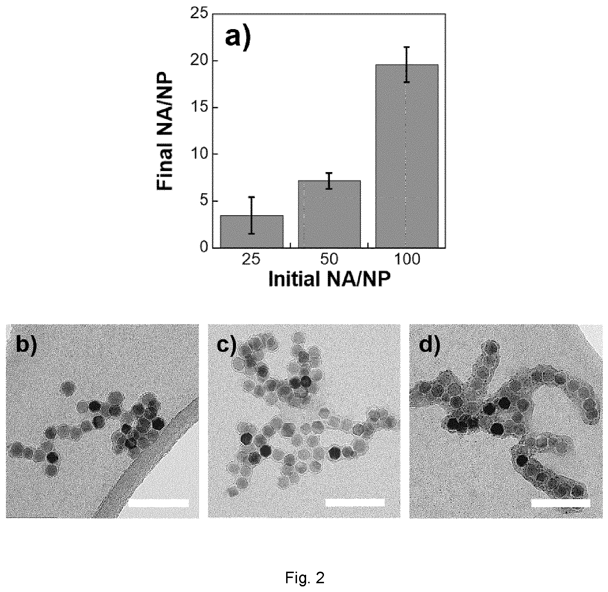

**Figure 2****.** a) Quantification of NA molecules per Fe₃O₄ nanoparticle by UV-Vis spectroscopy. Data represents the mean and standard deviation of three independent experiments. TEM micrographs of the samples: b) Fe₃O₄@NA₃, c) Fe₃O₄@NA₇, and d) Fe₃O₄@NA₂₀. The scale bar represents 100 nm.

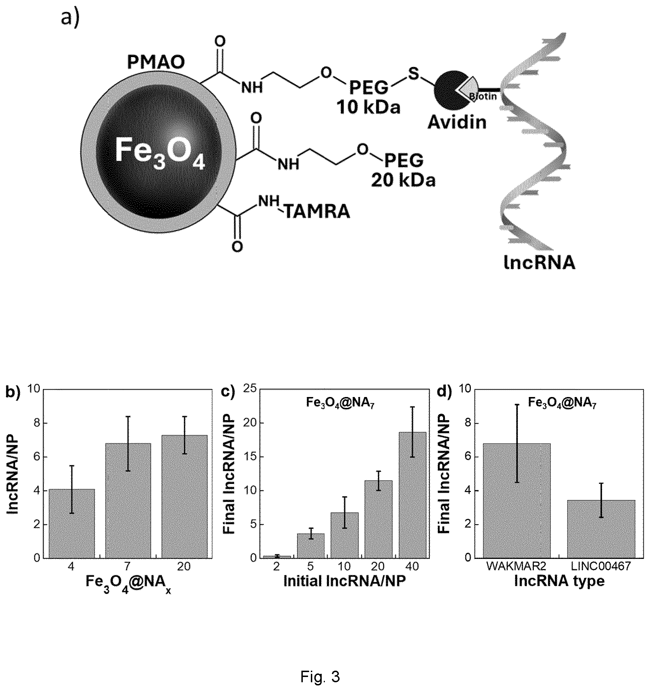

**Figure 3****.** a) Schematic drawing of Fe₃O₄@WAKMAR2 nanoplatform. Quantification plots of IncRNA loading per Fe₃O₄ nanoparticle (NP) via UV-Vis spectroscopy: b) Final WAKMAR2 per NP by increasing the NA/NP ratio and c) Final WAKMAR2 per NP by increasing the IncRNA/NP ratio and d) Final IncRNA/NP (WAKMAR2 and LINC00467) when using Fe₃O₄@NA₇ and an initial IncRNA/NP ratio of 10:1.

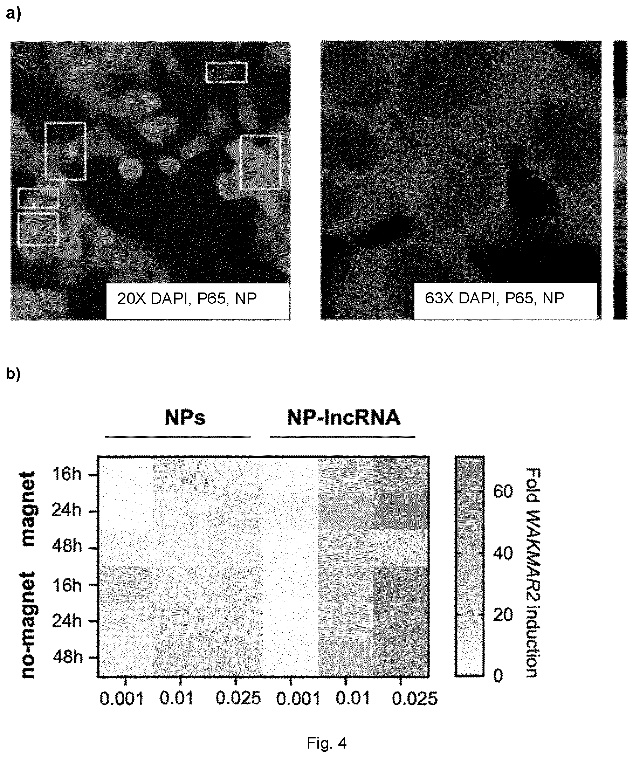

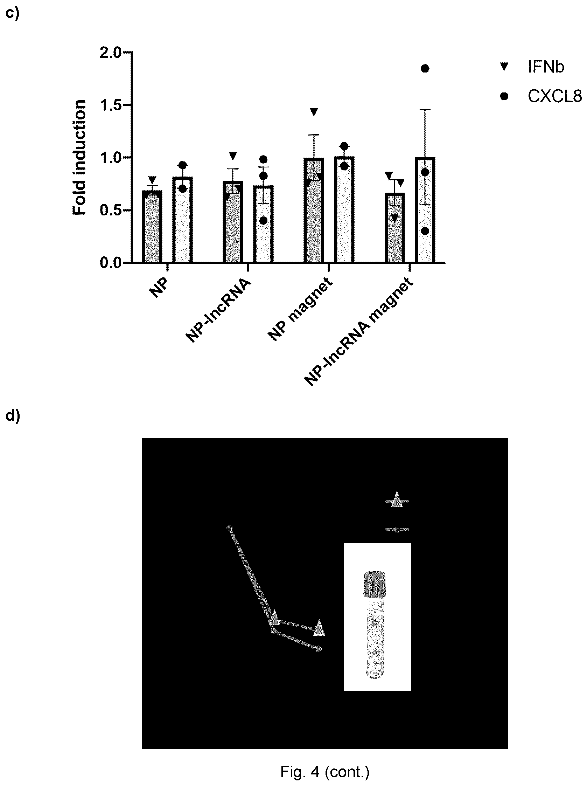

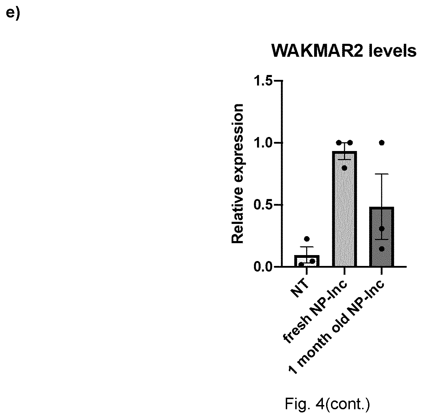

**Figure 4****.** a) confocal microscopy images at low (20x) (left) and at high (63x) (right) magnifications where it is seen how the NPs are introduced into HCT116 cells, b) WAKMAR2 expression in cells after adding NPs alone or NPs coupled with the IncRNA at different concentrations (with and without magnetic targeting), c) expression of inflammatory markers after incubation of cells with NPs and NPs-IncRNA complexes, d) WAKMAR2 stability after incubation in serum for 48 hours, e) In cell WAKMAR2 levels with a freshly made NP@IncRNA complex and with a complex kept at 4°C for a month.

### DETAILED DECRIPTION OF THE INVENTION

The inventors have developed new nanoparticles which have a long noncoding RNA attached which allows them to be used in diseases wherein the levels of long noncoding RNA are repressed or reduced. One major advantage of these nanoparticles is the fact that they comprise a magnetic core and an amphiphilic polymer outer shell, wherein the nanoparticles comprise long non-coding RNA (IncRNA), which make the nanoparticles highly versatile, as the inorganic core and the type of IncRNA can be adapted to specific healthcare demands.

### Nanoparticle of the invention

A first aspect of the present invention relates to a nanoparticle, from here onwards the nanoparticle of the invention, comprising a magnetic core and an outer shell wherein the outer shell comprises a long noncoding RNA.

The term "nanoparticle" as used herein refers to a particle having a diameter of between 1 nanometer (nm) and 1000 nm. In a particular embodiment, the nanoparticle of the invention has an average size of between 1 nm to about 10 nm, from about 10 nm to about 50 nm, from about 50 nm to about 100 nm, from about 100 nm to about 250 nm, from about 250 nm to about 500 nm, from about 500 nm to about 750 nm, or from about 750 nm to about 1000 nm. In a particular embodiment, the nanoparticle of the invention has an average size of between about 15 nm to about 25 nm, preferably an average size of about 18 nm to about 22 nm. In another particular embodiment the nanoparticle of the invention has an average size of about 10 nm, about 11 nm, about 12 nm, about 13 nm, about 14 nm, about 15 nm, about 16 nm, about 17 nm, about 18 nm, about 19 nm, about 20 nm, about 21 nm, about 22 nm, about 23 nm, about 24 nm, about 25 nm.

The "size" of a nanoparticle refers to the length of the largest straight dimension of the nanoparticle. For example, the size of a perfectly spherical nanoparticle is its diameter. The term "average size" refers to the measurement of a multitude of individual nanoparticles in a population of nanoparticles, leading to a distribution of sizes from which an average is obtained. Several techniques are known to measure the size of nanoparticles, such as, without limitation, dynamic light scattering, disc centrifugation, nanoparticle tracking analysis, tunable Resistive Pulse Sensing, atomic force microscopy, electron transmission microscopy and scanning electron microscopy. In a particular embodiment, the nanoparticle of the invention average size is measured by transmission electron microscopy.

It is to be understood that the nanoparticles according to the invention can have any shape, such as spherical, rhomboid, toroid, cubic, ovoid, triangular, branched, cylindrical, needle, hexagonal, pentagonal, rectangular, octahedral, etc., or can even lack a clear shape.

The nanoparticles of the present invention are characterized by comprising a magnetic core. As used herein, the term "magnetic core" refers to nanoparticles that comprises the central or innermost part of the nanoparticle, containing a material that has the ability to produce a magnetic field that can attract or repel certain substances. In the present invention, the term "magnetic core" includes paramagnetic core, diamagnetic core, and ferromagnetic core. The magnetic core endows the nanoparticle with magnetic properties, and therefore, the nanoparticle of the invention can also be defined as magnetic nanoparticle.

The term "paramagnetic" as used herein refers to a class of substances that when placed in a magnetic field are magnetized parallel to the line of force in the field and proportional to the intensity of the field.

The term "diamagnetic" as used herein refers to a class of substances that when placed in a magnetic field are repelled by the magnetic field.

The term "ferromagnetic material" means that a material has the property of becoming magnetized under the action of an external magnetic field, and remaining long magnetized when the field is canceled.

Examples of magnetic core materials, without limitation, of the nanoparticles of the invention, are iron oxides of composition Fe₂O₃, or Fe₃O₄, and ferrites of general composition MFe₂O₄ or MₓFe₃₋ₓO₄ wherein M is a divalent metal such as Cobalt (Co), Manganese (Mn) or Zinc (Zn) and x is the content of the dopant metal. Other suitable materials are γ-Fe₂O₃, the pure metals Co, Fe, Nickel (Ni), Zinc (Zn) and metal compounds such as carbides and nitrides. In a particular embodiment, the magnetic core is made of an iron oxide, or iron oxide doped with metal ions selected from Co²⁺, Mn²⁺, Zn²⁺, and Ni²⁺, or a combination thereof. In a particular embodiment, the magnetic core comprisesstoichiometric ferrites (MFe₂O₄). In a particular embodiment, the magnetic core is made of an iron oxide. In another particular embodiment, the iron oxide is selected from Fe₂O₃, and Fe₃O₄. In a further particular embodiment, the iron oxide is Fe₃O₄.

Due to their magnetic core, the magnetic nanoparticles according to the invention can be characterized by their magnetization properties, which refers to the vector field that expresses the density of permanent or induced magnetic dipole moments in the magnetic nanoparticles. Furthermore, the saturation magnetization of the nanoparticles of the invention can also be determined. The term "saturation magnetization" as used herein refers to a state reached by the nanoparticles wherein an increased in the applied external magnetic field cannot increase the magnetization of the nanoparticles further, so the total magnetic flux density levels off. In a particular embodiment, the nanoparticles of the invention have a saturation magnetization at room temperature of more than 75 A·m²/kg, more than 80 A. m²/kg, more than 85 A·m²/kg, more than 90 A·m²/kg, more than 95 A·m²/kg, preferably more than 90 A·m²/kg.

Another characteristic of the magnetic particles according to the invention is the "polydispersity index", which refers to the broadness of the nanoparticle size distribution, and can range from 0.0 (for a perfect uniform sample with respect to particle size) to 1.0 (for a highly polydisperse sample with multiple particle size populations). In a particular embodiment, the nanoparticles of the invention have a polydispersity index of less than 0.32, less than 0.3, less than 0.28.

A further characteristic of the magnetic particles according to the invention is the "hydrodynamic diameter", or "D_{H}", which refers to the diameter of a hard sphere that would experience the same drag force as the nanoparticles when moving in a liquid. In a particular embodiment, the nanoparticles of the invention have a hydrodynamic diameter of about between 65 nm and about 85 nm, preferably about 65 nm, about 66 nm, about 67 nm, about 68 nm, about 69 nm, about 70 nm, about 71 nm, about 72 nm, about 73 nm, about 74 nm, about 75 nm, about 76 nm, about 77 nm, about 78 nm, about 79 nm, about 80 nm, about 81 nm, about 82 nm, about 83 nm, about 84 nm, about 85 nm.

Yet another characteristic of the magnetic nanoparticles according to the invention, refers to their "Zeta potential", or "P_{Z}", which refers to the charge that develops at the interface between the nanoparticle surface and its liquid medium. In a particular embodiment, the nanoparticles of the invention have a zeta potential of about -15 millivolts (mV) or less, preferably about -15 mV, about -16 mV, about -17 mV, about -18 mV, about -19 mV, about -20 mV, about -21 mV, about -22 mV, about -23 mV, about -24 mV, about -25 mV.

The magnetic properties of the magnetic nanoparticles, including saturation magnetization, were studied using a Direct Current (DC) Superconducting Quantum Interference Device (SQUID) magnetometer (see Examples). This device allows the measurement of extremely weak magnetic fields, based on superconducting loops containing Josephson junctions. Further characteristics of the magnetic particles such as size distribution, zeta potential, hydrodynamic diameter, and polydispersity index were studied using dynamic light scattering (DLS) as mentioned in the examples.

The nanoparticles of the invention are further characterized by comprising an outer shell. The term "outer shell" as used herein refers to an outermost layer of the nanoparticle that substantially covers the underlying magnetic core. In a particular embodiment, the outer shell contains an amphiphilic polymer. In another particular embodiment, the outer shell comprises an amphiphilic polymer or consists of an amphiphilic polymer. The skilled person in the art will know of techniques to coat the magnetic core with amphiphilic polymers, such as the one exemplified in the examples in the present description.

The term "amphiphilic polymer" as used herein refers to a polymer having distinct polar (hydrophilic) and nonpolar (hydrophobic) regions or blocks. Such distinct polar and non-polar regions in the molecule promote the formation of micelles in dilute aqueous solutions. For example, an amphiphilic polymer may contain a large organic cation or anion, such as H₃C(CH₂)ₙ CO²⁻ or H₃C(CH₂)ₙ SO³⁻ or H₃C(CH₂)ₙ N(CH₃)³⁺, which possesses a long unbranched hydrocarbon chain, n>7.

In a particular embodiment, the amphiphilic polymer is selected from the group consisting of: poly(ethylene glycol) (PEG), poly(styrene-co-vinylpyrrolidone), poly(lauryl methacrylate), poly(oligo(ethylene glycol) methacrylate), poly(acrylic acid), poly(maleic anhydride-alt-1-octadecene) (PMAO), poly(N-vinylpyrrolidone) (PVP), poly(2-ethyl-2-oxazoline) (PEtOx), and combinations or block copolymers thereof with other hydrophilic or hydrophobic monomers, optionally functionalized for specific surface conjugation.

In a particular embodiment, the amphiphilic polymer is a copolymer. In another particular embodiment, the outer shell contains amphiphilic polymers and/or amphiphilic copolymers.

As used herein, the term "copolymer" refers to a polymer composed of two or more different monomer units. In a particular embodiment, the amphiphilic copolymer is selected from a group consisting of: polyethylene glycol-containing copolymers, polyethylene oxides, polyacrylamides, polyacrylic acids and their copolymers, acrylates, maleic anhydride copolymers, methacrylates, ethacrylates and related polymers, amine-functional polymers (e.g., polyethyleneimines, poly(2-ethyl-2-oxazoline), polyallylamines), ethers (e.g., polymethylvinyl ethers), polyepoxysuccinic acids, glycerol propoxylates, styrene-based copolymers (e.g., polystyrenesulfonate), vinyl alcohols (e.g., polyvinyl alcohol), vinyl acids, poly(vinylpyridines), poly(vinylpyrrolidones), block copolymers (e.g., Pluronics^{®}, PEG-PLGA, PEG-PCL), polysaccharide-based copolymers (e.g., chitosan, dextran derivatives), and zwitterionic copolymers (e.g., PSBMA, PCBMA).

In another particular embodiment the amphiphilic copolymer is poly(lauryl methacrylate)-b-poly(oligo ethylene glycol methacrylate) and/or poly(maleic anhydride-alt-1-octadecene)-poly(ethylene glycol) (PMAO-PEG).

In another particular embodiment the amphiphilic copolymer is poly(maleic anhydride-alt-1-octadecene)-poly(ethylene glycol) (PMAO-PEG).

In the present invention, the amphiphilic polymers which constitute the outer shell of the nanoparticle of the invention can be functionalized with a molecule which is able to bind to another molecule present in the long noncoding RNA.

In a particular embodiment, the amphiphilic polymer is functionalized first with a member of a binding pair of molecules. In the context of the present invention, the expression "is functionalized with a first member of a binding pair of molecules" refers to the fact that the amphiphilic polymer and/or copolymer of the outer shell of the nanoparticles comprises a functional group or a molecule, the "first member", which is bound to the polymer and/or copolymer, such that said functional group or molecule is in the surface of the nanoparticle and allows for the binding to the another molecule, the "second member", being that both first and second members have specific binding affinity for each other, forming a "binding pair or molecules" allowing the polymers of the outer shell to bind to the IncRNA. Several techniques are known in the field that allow the functionalization of a molecule with another one, and one such technique is described in the Examples section of this description.

The functionalization of the outer shell of the nanoparticles of the invention, results in a set number of first members of the binding pair of molecules that is present in the surface of the nanoparticle, which is dependent on the functionalization procedure. In a particular embodiment, the nanoparticle of the invention comprises between 1 to about 20 first members of the binding pair of molecules. In another particular embodiment, the nanoparticle of the invention comprises between 1 to about 10 first members of the binding pair of molecules. In another particular embodiment, the nanoparticle of the invention comprises between 3 to about 5 first members of the binding pair of molecules. In another particular embodiment, the nanoparticle of the invention comprises about 1, about 2, about 3, about 4, about 5, about 6, about 7, about 8, about 9, about 10, about 11, about 12, about 13, about 14, about 15, about 16, about 17, about 18, about 19, about 20, or more first members of the binding pair of molecules.

The term "binding pair of molecules" in the present context refers to an attractive interaction between two molecules that results in a stable association in which the molecules are in close proximity to each other. Several types of molecule association can be used in order to achieve such binding, such as proteins, nucleic acids, carbohydrates, lipids, and small organic molecules such as drugs, resulting in different types of association. Examples of pairs of binding molecules are, without limitation, antibody and antigen, enzyme and substrate, receptor and ligand, DNA and DNA-binding proteins, protein and protein, calmodulin and calcium ions, cytokines and their receptors. Further examples of binding pairs of molecules are, without limitation, avidin and biotin, calmodulin and calcium ions.

In a particular embodiment, the first member of the binding pair of molecules is an avidin protein. In the present context, the term "avidin" indicates a group of proteins which are characterized, from a functional point of view, by the property that they bind biotin (also known as vitamin H and vitamin B7) with a very high affinity and specificity. They are produced by various higher organisms (birds, amphibians, etc.), wherein they are present in the eggs, or by a *Streptomyces* (*Streptomyces avidinii*)*.* The former group of proteins are strongly basic glyco-proteins known as avidin in the strict sense; the latter is not a glycoprotein, has an isoelectric point near neutrality, and is known by the term of streptavidin. These proteins are commercially available either in the native form or modified as a result of partial proteolysis (like the so-called core streptavidin), partial deglycosylation or chemical modification (acetylavidin, succinylavidin, etc.).

In a particular embodiment, the avidin protein is selected form a group consisting of: streptococcus avidin (streptavidin), chicken avidin (extravidin), neutralite avidin (neutravidin), core streptavidin. In another particular embodiment, the aviding protein is deglycosylated.

The term "deglycosylated" refers to the process of "deglycosylation", which in the present context refers to the removal of carbohydrate groups from the avidin protein, which can enhance its binding affinity and reduce potential immunogenicity. This modification can lead to improved biocompatibility and reduced immune response when the nanoparticle is introduced into a biological system. Additionally, deglycosylated avidin may exhibit increased stability and reduced steric hindrance, allowing for more efficient interactions with biotin or biotinylated molecules.

As previously stated, the binding pair of molecules comprises a first member in the outer shell of the nanoparticle and a second member, which is bound to the long noncoding RNA. In another particular embodiment, the long noncoding RNA is functionalized with a second member of the binding pair of molecules. In a further particular embodiment, the long noncoding RNA is functionalized with a second member of the binding pair of molecules which is bound to the first member of the binding pair of molecules

In a particular embodiment, the second member of the binding pair of molecules is biotin and the first member of the binding pair is avidin as previously described herein.

The term "biotin" as used herein, refers to D-biotin, an essential water-soluble vitamin also known as Vitamin H, Coenzyme R, or vitamin B7. D-Biotin has Chemical Abstracts Service Registry No. 58-85-5. The term biotin as used herein also includes the term "biotin salt", which refers to an organic or inorganic salt of D-biotin. Typical salts include alkali metal, alkaline earth metal, ammonia, or organic amine salts as, for example, sodium, potassium, magnesium, calcium, protonated amines such as those derived from ethylamine, triethylamine, ethanolamine, diethylamino-ethanol, ethylenediamine, piperidine, morpholine, 2-piperidinoethanol, benzylamine, procaine and the like.

Other means of binding the outer shell polymers to the long noncoding RNA are known by the skilled person in the art. In particular, another means refers to click chemistry. The term "click chemistry" as used herein refers to chemistry tailored to generate covalent bonds quickly and reliably by joining small units comprising reactive groups together. Click chemistry does not refer to a specific reaction, but to a concept including, but not limited to, reactions that mimic reactions found in nature. Click chemistry reactions can be modular, wide in scope, give high chemical yields, generate inoffensive byproducts, are stereospecific, exhibit a large thermodynamic driving force to favor a reaction with a single reaction product, and/or can be carried out under physiological conditions. In certain embodiments, a click chemistry reaction exhibits high atom economy, can be carried out under simple reaction conditions, use readily available starting materials and reagents, uses no toxic solvents or uses a solvent that is benign or easily removed (preferably water), and/or provides simple product isolation by non-chromatographic methods (crystallization or distillation).

In a particular embodiment, the first member of the binding pair of molecules is a handle of a pair of partner click chemistry handles and the second member of the binding pair of molecules is a second handle of the pair of partner click chemistry handles, wherein the pair of partner click chemistry handles are reactive with each other, and wherein both handles are interchangeable between the first member and the second member.

The term "click chemistry handle" refers to a reactant, or a reactive group, that can partake in a click chemistry reaction. For example, a strained alkyne, e.g., a cyclooctyne, is a click chemistry handle, since it can partake in a strain-promoted cycloaddition. In general, click chemistry reactions require at least two molecules comprising click chemistry handles that can react with each other. Such click chemistry handle pairs that are reactive with each other are sometimes referred to as partner click chemistry handles. For example, an azide is a partner click chemistry handle to a cyclooctyne or any other alkyne. In a particular embodiment, the pair of partner click chemistry handles is a conjugated diene and a substituted alkene. In other embodiments, the pair of partner click chemistry handles are substituted tetrazine (Tz) and substituted trans-cyclooctene (TCO). Tz and TCO react with each other in a reverse-electron demand Diels-Alder cycloaddition reaction. In other embodiments, the pair of partner click chemistry handles are substituted alkyne and substituted azide. For example, a difluorinated cyclooctyne, a dibenzocyclooctyne, a biarylazacyclooctynone, or a cyclopropyl-fused bicyclononyne can be paired with an azide as a pair of partner click chemistry handles. In other embodiments, the pair of partner click chemistry handles are reactive dienes and suitable tetrazine dienophiles. For example, TCO, norbornene, or biscyclononene can be paired with a suitable tetrazine dienophile as a click chemistry pair. In yet other embodiments, the pair of partner click chemistry handles are tetrazoles and unactivated alkenes, which are reactive in the presence of ultraviolet light. This is termed a "photo-click" chemistry pair. In another embodiment, the pair of partner click chemistry handles is a cysteine and a maleimide. For example, the cysteine from a peptide (e.g., GGGC) may be reacted with a maleimide. Other suitable click chemistry handles are known to those of skill in the art (Spicer et al., "Selective chemical protein modification." Nature Communications. 2014; 5:4740). For two molecules to be conjugated via click chemistry, the click chemistry handles of the molecules have to be reactive with each other, for example, in that the reactive moiety of one of the click chemistry handles can react with the reactive moiety of the second click chemistry handle to form a covalent bond. Such reactive pairs of click chemistry handles are well known to those of skill in the art.

In a particular embodiment of the nanoparticle of the invention, wherein the magnetic core is iron oxide Fe₃O₄, the outer shell contains a PMAO-PEG copolymer wherein the molecules of PMAO-PEG copolymer are functionalized with avidin protein and wherein the long noncoding RNA is functionalized with biotin and the biotin is bound to the avidin protein.

As previously mentioned, the nanoparticle of the invention is characterized by comprising a long noncoding RNA.

The term "long non coding RNA", also referred to as "long noncoding RNA", "long non-coding RNA", and "IncRNA", in the present context refers to a functional RNA molecule that is not translated into a protein. The DNA sequence from which a non-coding RNA is transcribed is often called in the art an RNA gene. The term "IncRNA" or "long non-coding RNA" as commonly used in the art designates a noncoding RNA comprising more than 200 nucleotides. LncRNAs can interact with DNA, RNA, and proteins. LncRNAs can be classified as: Signal IncRNAs, which are specifically associated with signaling pathways and regulate downstream gene transcription; decoy IncRNAs, which interact with transcription factors and remove them from chromatin, thereby influencing transcriptional regulation; guide IncRNAs, which bind to protein complexes with regulatory effects or enzymatic activities and direct them to target gene promoters or specific genomic sites to regulate downstream signaling events and gene expression; scaffold IncRNAs, a 'central platform' connecting various protein complexes, which are directed to a specific genomic location or target gene promoter region to regulate gene expression. In addition to this, LncRNA can participate in the regulation of a variety of biological processes, including but not limited to, RNA processing, miRNA sponge (ceRNA), mRNA stability, transcriptional regulation, translational regulation, splicing regulation, port-translational regulation.

The term "IncRNA" is meant to include all forms and types of IncRNAs, in particular, intergenic IncRNAs - transcribed intergenically from both strands, intronic IncRNAs - transcribed entirely from introns of protein-coding genes, sense IncRNAs - transcribed from the sense strand of protein-coding genes and contain exons from protein-coding genes, , overlapping with part of protein-coding genes or covering the entire sequence of a protein-coding gene through an intron, and antisense IncRNAs, transcribed from the antisense strand of protein-coding genes, overlapping with exonic or intronic regions or covering the entire protein-coding sequence through an intron, as well as cis-acting IncRNAs - (cis-acting IncRNAs) that regulate the expression of genes in close genomic proximity, and trans-IncRNAs - (trans-acting IncRNAs) that regulate the expression of distant genes. Furthermore, the IncRNAs according to the present invention further include IncRNAs which function as signaling IncRNAs, those associated with specific signaling pathways and their expression indicates an active signaling event, decoy IncRNAs, those that act as "sponges" for miRNAs, preventing them from binding to their target mRNAs, guide IncRNAs, those that guide proteins to specific locations in the cell, and scaffold IncRNAs, those that act like platforms for protein-protein interaction.

The IncRNAs as described in the present invention also include naturally and artificially modified IncRNAs, in particular without limitation, IncRNAs as describe herein include IncRNAs comprising one or more modifications in their sequence, wherein the modifications are selected from: N6-methyladenosine (m6A), 5-methylcytosine (m5C), N1-methyladenosine (m1A), N7-methylguanosine (m7G) and pseudouracil (Ψ).

In the context of the present invention, the long noncoding RNA or its nature is not limiting to the nanoparticle of the invention. In a preferred embodiment of the nanoparticle of the invention, the long noncoding RNA is the product of the transcription of one of the genes described in Table 1.

**Table 1: LncRNAs associated with disease development. The database entry in the RNA central database or sequence identifier is provided for every IncRNA.**

| **LncRNA gene** | **Treatment** | **Database accession number - RNAcentral (https://rnacentral.org/) or scientific publication** |
|---|---|---|
| LOC339803 | Inflamatory bowel Disease | URS000075B239_9606 |
| CHASER | Neurodevelopmental disorder | URS000075A1CF 9606 |
| Swhtr | Myocardial injury | Rogala S., et al. Nature Communications, 2023, 7024 |
| HOXA11-AS1 | Glioma | URS00007333DC_9606 |
| LlNC01257 | Acute Myeloid Leukemia | URS000075B626 9606 |
| HOTTIP | Rheumatoid Arthritis | URS000075E1E7 9606 |
| IncOb | Obesity | Dallner O.S., et al., Nature Medicine, 2019, 25(3):507-516 |
| Inc13 | Celiac Disease | URS0000EBA19C_9606 |
| FOXF1 adjacent non-coding developmental regulatory RNA (Fendrr) | Cardiovascular disease | URS0000EEF7EA_9606 |
| Kcnq1ot1 | Cardiovascular disease | URS00000CE0D1_9606 |
| H19 imprinted maternally expressed transcript | Cardiovascular disease /type 2 diabetes | URS0000812128_9606 |
| non-protein coding Inc-FA2H-2 | atherosclerosis / inflammation | URS0000D6E7C7_9606 |
| Homo sapiens (human) non-protein coding Inc-CCL2-2:4 | atherosclerosis / inflammation | URS00008BA46C_9606 |
| HIF1A antisense RNA 2 (HIF1A-AS2) | atherosclerosis | URS000075BB37_9606 |
| AK136714 | Inflammation | |
| MALAT1 | Inflammation/ Parkinson's disease (PD) | URS00008C0A2F_9606 |
| nuclear paraspeckle assembly transcript 1 (NEAT1) | Inflammation/ Diabetic retinopathy | URS000075DAEC_9606 |
| CDKN2B antisense RNA 1 (CDKN2B-AS1) | Cardiovascular disease | URS00026A21AD_9606 |
| MAP3K4 antisense RNA 1 (MAP3K4-AS1) | Inflammation | URS000008AA49_9606 |
| NEXN antisense RNA 1 (NEXN-AS1) | Inflammation | URS000032A8EE_9606 |
| ENST00000416361 | Cardiovascular disease | |
| deleted in lymphocytic leukemia 1 (DLEU1) | osteoarthritis (OA) / Inflammation | URS000075DA5A_9606 |
| LOXL1 antisense RNA 1 (LOXL1-AS1) | osteoarthritis (OA) / Inflammation | URS000075AECF_9606 |
| long intergenic non-protein coding RNA 265 (LINC00265) | osteoarthritis (OA) / Inflammation | URS000075CC82_9606 |
| Prader Willi/Angelman region RNA 5 (PWAR5) | osteoarthritis (OA) / Inflammation | URS000075990E_9606 |
| HOX transcript antisense RNA (HOTAIR) | osteoarthritis (OA) / Inflammation/cancer metastasis | URS00026A23F2_9606 |
| maternally expressed 3 (MEG3) | osteoarthritis (OA) / Inflammation/ respiratory inflammatory diseases | URS0000759EA9_9606 |
| nuclear paraspeckle assembly transcript 1 (NEAT1) | osteoarthritis (OA) / Inflammation | URS000075DAEC_9606 |
| NF-kappaB interacting IncRNA (NKILA) | osteoarthritis (OA) / Inflammation | URS00008120E1_9606 |
| MGAT3 antisense RNA 1 (MGAT3-AS1) | osteoarthritis (OA) / Inflammation | URS00001B3E73_9606 |
| non-protein coding TUG1 | osteoarthritis (OA) / Inflammation/sepsis/ Parkinson's disease (PD) | URS0000D5A0BF_9606 |
| LOC101927514 | respiratory inflammatory diseases | |
| SLC44A3 antisense RNA 1 | respiratory inflammatory diseases | URS00004BEF4B_9606 |
| small nucleolar RNA host gene 5 (SNHG5) | respiratory inflammatory diseases | URS000075B42D_9606 |
| small nucleolar RNA host gene 16 (SNHG16) | respiratory inflammatory diseases | URS000075DE45_9606 |
| GAS5 antisense RNA 1 (GAS5-AS1) | respiratory inflammatory diseases | URS000075DC2A_9606 |
| HLA complex P5 (HCP5) | Diabetic retinopathy | URS000075AF59_9606 |
| OGRU | Diabetic retinopathy | |
| KCNQ1 opposite strand/antisense transcript 1 (KCNQ1OT1) | Diabetic retinopathy | URS00000CE0D1_9606 |
| small nucleolar RNA host gene 7 (SNHG7) | Parkinson's disease (PD) | URS000075E30B_9606 |
| wound and keratinocyte migration associated IncRNA 2 (WAKMAR2) | Inflammation/cancer/chronic wounds | URS000075B835_9606 |

In a particular embodiment of the nanoparticle of the invention, the long noncoding RNA is the product resulting from the transcription of the gene WAKMAR2, accessible from the database NCBI Reference Sequence, with accession number NR_049793.1, entry of March 25, 2023.

The term "WAKMAR2", also known by "wound and keratinocyte migration associated IncRNA 2", as used herein refers to a gene whose product of transcription is not translated into a protein. The gene is associated with several biological processes, including being associated with wound healing and chronic wounds, as well as cancer development and inflammation. The gene is characterized by the sequence according to the database NCBI Reference Sequence, with accession number NR_049793.1, entry of March 25, 2023.

In a particular embodiment of the nanoparticle of the invention, the long noncoding RNA has a nucleotide sequence comprising at least 90% identity to SEQ ID NO: 1, or a functionally equivalent thereof. In another particular embodiment of the nanoparticle of the invention, the long noncoding RNA has a nucleotide sequence comprising at least 91%, 92%, 93%, 94%, 95%, 96%, 97%, 98%, 99%, 100% identity to SEQ ID NO: 1, or a functionally equivalent thereof. In a further embodiment of the nanoparticle of the invention, the long noncoding RNA has a nucleotide sequence which consists of SEQ ID NO: 1.

The terms "identity", "identical" or "percent identity" in the context of two or more amino acid or nucleotide sequences, refer to two or more sequences or subsequences that are the same or have a specified percentage of nucleotide or amino acid residues that are the same, when compared and aligned (introducing gaps, if necessary) for maximum correspondence, not considering any conservative substitutions as part of the sequence identity. The percent identity can be measured using sequence comparison software or algorithms or by visual inspection. Various algorithms and software are known in the art that can be used to obtain alignments of amino acid or nucleotide sequences, such as, the Needleman-Wunsch algorithm and the Smith-Waterman algorithm. Publicly available software programs can be used to align sequences, such as, EMBOSS Needle (https://www.ebi.ac.uk/jdispatcher/psa/emboss_needle) and NIH Basic Local Alignment Search Tool (BLAST) (https://blast.ncbi.nim.nih.gov/BlastAlign.cgi). Appropriate parameters for maximal alignment by particular alignment software can be determined by one skilled in the art. In certain embodiments, the default parameters of the alignment software are used. In certain embodiments, the percentage identity "X" of a first nucleotide sequence to a second nucleotide sequence is calculated as 100 x (Y/Z), where Y is the number of nucleotide residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length which is substantially the same as the length of the first sequence.

For instance, 95% identical to a reference sequence according to the present invention, the parameters are set such that the percentage of identity is calculated over the full length of the reference nucleotide sequence and that gaps in homology of up to 5% of the total number of nucleotides in the reference sequence are allowed.

In another particular embodiment of the nanoparticle of the invention, the long noncoding RNA sequence identity is calculated over the whole length of both sequences being compared. In another particular embodiment of the nanoparticle of the invention, the long noncoding RNA sequence identity is calculated using the Needleman-Wunsch algorithm.

As it is used herein, the term "functionally equivalent variant thereof" refers to a sequence variant of SEQ ID NO: 1, which encodes for WAKMAR2, said sequence variant having substantially similar sequence identity with it and substantially maintaining its capacity to induce inflammation markers, such as CXCL8, when integrated into cells on the nanoparticles of the invention. For example, a functionally equivalent variant of SEQ ID NO: 1 may be a polypeptide sequence derivative of said sequence comprising the addition, deletion or substitution of one or more amino acids.

In another particular embodiment of the nanoparticle of the invention, the nanoparticle further comprises a marker bound to the outer shell.

The term "markers", used interchangeably with the terms "labels" or "tags", as used herein refer to components that can be attached to the outer shell and may allow the localization of the nanoparticles or may improve or facilitate certain properties of the nanoparticle or its constituents, i.e. IncRNA stability or resistance to degradation, or provide technical advantages during production, i.e., facilitate production. Examples of such markers/labels/tags are, without limitation, fluorescent agent or a fluorochrome (dye), such as fluorescein isocyanate (FIC), fluorescein isothiocyante (FITC), 5-dimethylamine- 1-natpthalenesulfonyl chloride (DANSC), tetramethylrhodamine isothiocyanate (TRITC), lissamine, rhodamine 8200 sulphonyl chloride (RB 200 SC), 5-TAMRA cadaverine, biotin, His tag, FLAG tag, Halo tag, MBP tag, HA tag, Myc tag, V5 tag, PA tag, fluorescent protein tag and the like. Said tags can be functionalized to the outer shell components by methods previously explained, or it can be present in the long noncoding RNA present in the outer shell by modification of RNA sequence.

### Methods of the invention

All embodiments and definitions previously described are equally valid and applicable for the aspects of the invention relating to methods.

The nanoparticle of the invention can be obtained by combining a magnetic core with a surrounding layer, which will form the outer shell, and placing a long noncoding RNA in the outer shell. Hence, another aspect of the present invention relates to a method for obtaining a nanoparticle of the invention, from here onwards the method I of the invention, comprising the steps of:
a) providing a nanoparticle comprising a magnetic core and an outer shell wherein the outer shell is functionalized with a first member of a binding pair of molecules;
b) providing a long noncoding RNA functionalized with a second member of the binding pair of molecules;
c) bringing the nanoparticle of step a) into contact with the long noncoding RNA of step b) in conditions such that the second member of the binding pair of molecules binds to the first member of a binding pair of molecules.

In a particular embodiment of method I of the invention, the magnetic core is made of iron oxide. In a more particular embodiment of method I of the invention, the iron oxide is selected from Fe₂O₃, and Fe₃O₄. In an even more particular embodiment of method I of the invention, the iron oxide is Fe₃O₄.

In another particular embodiment of method I of the invention, the outer shell contains an amphiphilic polymer. In a further particular embodiment of method I of the invention, the amphiphilic polymer is selected from a group consisting of: poly (ethylene glycol), poly(styrene-co-vinylpyrrolidone), poly(lauryl methacrylate), poly(oligo ethylene glycol methacrylate), poly(acrylic acid), poly(maleic anhydride-alt-1-octadecene), and any combination thereof.

In another particular embodiment of method I of the invention, the amphiphilic polymer is a copolymer. In another particular embodiment of method I of the invention, the outer shell contains amphiphilic polymers and/or amphiphilic copolymers.

In a particular embodiment of method I of the invention, the amphiphilic copolymer is selected from a group consisting of: polyethylene glycols, polyethylene oxides, polyacrylamides, polyacrylic acids and their related polymers and co-polymers, acrylates, maleic anhydride copolymers, methacrylate, ethacrylate and related polymers, amine-functional polymers, such as polyethyleneimines, poly-2-ethyl-2-oxazolines and polyallylamines, ethers, such as polymethylvinyl ethers, polyepoxysuccinic acid, glycerol propoxylates, styrenes, such as polystyrenesulfonate and related polymers, vinyl acids, and vinyl alcohols, such as polyvinyl alcohols, poly(vinylpyridines) and poly(vinylpyrrolidone).

In another particular embodiment of method I of the invention, the amphiphilic copolymer is poly(lauryl methacrylate)-b-poly(oligo ethylene glycol methacrylate) and/or poly(maleic anhydride-alt-1-octadecene)-poly(ethylene glycol) (PMAO-PEG), preferably poly(maleic anhydride-alt-1-octadecene)-poly(ethylene glycol) (PMAO-PEG).

In a particular embodiment of method I of the invention, the amphiphilic polymer is functionalized with a first member of a binding pair of molecules. In a particular embodiment of method I of the invention, the first member of the binding pair of molecules is an avidin protein. In a particular embodiment of method I of the invention, the avidin protein is selected form a group consisting of: streptococcus avidin (streptavidin), chicken avidin (extravidin), neutralite avidin (neutravidin), core streptavidin. In another particular embodiment of method I of the invention, the aviding protein is deglycosylated.

In a further particular embodiment of method I of the invention, the long noncoding RNA is functionalized with a second member of the binding pair of molecules, which is bound to the first member of the binding pair of molecules. In a particular embodiment of method I of the invention, the second member of the binding pair of molecules is biotin and the first member of the binding pair is avidin

In a particular embodiment of method I of the invention, the first member of the binding pair of molecules is a handle of a pair of partner click chemistry handles and the second member of the binding pair of molecules is a second handle of the pair of partner click chemistry handles, as previously described, wherein the pair of partner click chemistry handles are reactive with each other, and wherein both handles are interchangeable between the first member and the second member.

In a preferred embodiment of method I of the invention, the long noncoding RNA is the product of the transcription one of the genes described in Table 1.

In a particular embodiment of method I of the invention, the long noncoding RNA is the product resulting from the transcription of the gene WAKMAR2, accessible from the database NCBI Reference Sequence, with accession number NR_049793.1, entry of March 25, 2023. In a particular embodiment of method I of the invention, the long noncoding RNA has a nucleotide sequence comprising at least 90% identity to SEQ ID NO: 1, or a functionally equivalent thereof. In another particular embodiment method I of the invention, the long noncoding RNA has a nucleotide sequence comprising at least 91%, 92%, 93%, 94%, 95%, 96%, 97%, 98%, 99%, 100% identity to SEQ ID NO: 1, or a functionally equivalent thereof. In a further embodiment of method I of the invention, the long noncoding RNA has a nucleotide sequence which consists of SEQ ID NO: 1.

In order to modulate the amount of long noncoding RNA present in the nanoparticles, two strategies can be followed: i) increasing the amount of the first member of the binding pair while maintaining constant the initial ratio of long noncoding RNA to nanoparticles (IncRNA-to-NP ratio); or ii) maintaining the first member of the binding pair to nanoparticles (fBP-to-NP) ratio constant while increasing the initial IncRNA-to-NP ratio. In a particular embodiment of method I of the invention, step c) is carried out with a ratio of first member of the binding pair to nanoparticles (fBP-to-NP) higher than the ratio of long noncoding RNA to nanoparticles (IncRNA-to-NP). In another particular embodiment of method I of the invention, step c) is carried out with a ratio of first member of the binding pair to nanoparticles (fBP-to-NP) lower than the ratio of long noncoding RNA to nanoparticles (IncRNA-to-NP).

The term "ratio" as used herein refers to the quantitative relation between two amounts showing the number of times one value contains or is contained within the other.

Another aspect of the present invention relates to a method for obtaining a nanoparticle according to the invention, from here onwards method II of the invention, wherein the magnetic core is iron oxide Fe₃O₄, the outer shell contains a PMAO-PEG copolymer wherein the molecules of PMAO-PEG copolymer are functionalized with avidin protein and wherein the long noncoding RNA is functionalized with biotin and the biotin is bound to the avidin protein, wherein the nanoparticle is obtained by a method comprising the steps of:
a) providing a suspension comprising the Fe₃O₄ magnetic core;
b) providing a solution comprising the PMAO-PEG copolymer;
c) mixing the suspension of step a) and the solution of step b) such that the PMAO-PEG copolymer binds to and coats the Fe₃O₄ magnetic core, obtaining nanoparticles;
d) functionalizing the PMAO-PEG copolymer of the nanoparticles obtained in step c) with avidin protein;
e) bringing into contact the nanoparticles obtained in step d) with a long noncoding RNA functionalized with biotin, such that the biotin binds to the avidin protein.

### First step of method II

In the first step of method II a suspension comprising the magnetic core is provided. The term "suspension" as used herein refers to a heterogeneous fluid of an internal phase (i.p.) that is a solid and an external phase (e.p.) that is a liquid. In the present context the internal phase is the Fe₃O₄ magnetic core and the external phase the liquid into which the magnetic core is suspended, e.g. water.

In a particular embodiment of method II of the invention, the suspension comprising the magnetic core of step a) is obtained by a method comprising the following steps:
i) dissolving iron (III) oleate in a mixture of 1-octadecene and dibenzyl ether with oleic acid obtaining a mixture;
ii) heating the mixture of step i) at 10°C/min from room temperature up to 190°C;
iii) heating the heated mixture of step ii) at 3°C/min from 190°C to 330°C, allowing reflux for at least 60 min;
iv) allow the mixture of step iii) to cool to room temperature, obtaining a suspension with the magnetic core
wherein all steps i)-iv) are carried out under mechanical stirring.

In a particular embodiment of the method to obtain the suspension comprising the magnetic core of step a) of method II of the invention, the concentration of FeOl in step i) is 0.25 mmol/mL, the mixture of 1-octadecene and dibenzyl ether is at a ratio of 1:2 of 1-octadecene to dibenzyl ether and the concentration of oleic acid is of 0.5 mmol/mL.

Steps ii) and iii) of the method to obtain the suspension comprising the magnetic core of step a) of method II of the invention refer to the heating of the mixture of step i) and the heated mixture of step ii). The heating is done by any available and applicable heat source. The term "reflux" as used herein refers to the process of boiling a liquid so that any vapour is liquefied and returned to the stock.

The term "mechanical stirring" means that a magnetic device inside said reactor, such as for example a magnetic stirring bar, it is not used. Examples of mechanical stirring methods are, without limitation, rotation and vortexing. In another particular embodiment of method II of the invention, the mechanical stirring is done by rotation at 120 rotations per minute (rpm).

In a particular embodiment of the method to obtain the suspension comprising the magnetic core of step a) of method II of the invention, the mixture obtained in step iv) is further cleaned by centrifugation using tetrahydrofuran and ethanol at a temperature of less or equal to 6 °C.

### Second step of method II

In the second step of method II a suspension comprising the PMAO-PEG copolymer is provided. In a particular embodiment of method II of the invention, the solution comprising the PMAO-PEG copolymer of step b) of method II of the invention is obtained by a method comprising the steps of:
i) adding amine-poly(ethylene glycol)-thiol to a solution of poly(maleic anhydride-alt-1-octadecene) in an immiscible solvent and stirring the obtained mixture at room temperature overnight;
ii) adding poly(ethylene glycol)-amine to the mixture obtained in ii) and stirring the obtained mixture at room temperature for at least 12h, preferably at least 24h, obtaining the PMAO-PEG copolymer.

In a particular embodiment of the method for obtaining the solution comprising the PMAO-PEG copolymer of step b) of method II of the invention, the solution of poly(maleic anhydride-alt-1-octadecene) in step i) is at a concentration of 10 mg/ml. In another particular embodiment of the method for obtaining the solution comprising the PMAO-PEG copolymer of step b) of method II of the invention, the amine-poly(ethylene glycol)-thiol is added in step i) at a molar ratio of 5-75% of amine-poly(ethylene glycol)-thiol to poly(maleic anhydride-alt-1-octadecene), preferably at a molar ratio of 15-35% of amine-poly(ethylene glycol)-thiol to poly(maleic anhydride-alt-1-octadecene), more preferably at a molar ratio of 20% of amine-poly(ethylene glycol)-thiol to poly(maleic anhydride-alt-1-octadecene).

The term "immiscible solvent" as used herein refers to solvents that are incapable of mixing with another solvent and will separate out into a definite layer. Immiscible solvents cannot blend into or attain homogeneity with another liquid. Examples of immiscible solvents are, without limitation, acetone, acetonitrile, carbon tetrachloride, chloroform, cyclohexane, 1,2-dichloroethane, dichloromethane, diethyl ether, dimethyl formamide, dimethyl sulfoxide, 1,4-dioxane, ethanol, ethyl acetate, heptane, hexane, methanol, methyl-tert-butyl ether, pentane, 1-propanol, 2-propanol, tetrahydrofuran, toluene, 2,2,4-trimethylpentane, an water. In a particular embodiment of the method for obtaining the solution comprising the PMAO-PEG copolymer of step b) of method II of the invention, the immiscible solvent is chloroform.

The term "room temperature" as used herein refers to the known concept of a temperature of between about 20°C to about 25°C.

In a particular embodiment of the method for obtaining the solution comprising the PMAO-PEG copolymer of step b) of method II of the invention, the method further comprises an intermediate step after step i) and before step ii) of adding 5-TAMRA cadaverine to the mixture obtained in step i) and stirring the obtained mixture at room temperature overnight. In a further particular embodiment of the method for obtaining the solution comprising the PMAO-PEG copolymer of step b) of method II of the invention, the 5-TAMRA cadaverine is added at a concentration of between 5-35% w/v, preferably at a concentration of 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35% w/v, more preferably at a concentration of 10% w/v.

In a particular embodiment of the method for obtaining the solution comprising the PMAO-PEG copolymer of step b) of method II of the invention, step ii) is carried out for at least 12h, 13h, 14h, 15h, 16h, 17h, 18h, 19h, 20h, 21h, 22h, 23h, 24h or more hours.

In a particular embodiment of the method for obtaining the solution comprising the PMAO-PEG copolymer of step b) of method II of the invention, the poly(ethylene glycol)-amine is added in step ii) at a concentration of 75% w/v.

### Third step of method II

The third step of method II of the invention refers to the mixing of the solutions provided in the previous two steps, such that the PMAO-PEG copolymer coats the Fe₃O₄ magnetic core, obtaining nanoparticles. In a particular embodiment of the method II of the invention, step c) is carried out by a method comprising the following steps:
i) stepwise adding the suspension comprising the magnetic cores to the solution of comprising the outer shell until the suspension and solution are fully mixed;
ii) evaporating all the solvent of the mixture obtained in step ii) obtaining a solid;
iii) redispersing the solid obtained in step iii) in a buffer solution, obtaining a nanoparticle comprising a magnetic core and an outer shell.

In a particular embodiment of the method to carry out step c) of the method II of the invention, the stepwise addition is done by adding a drop at a time of the solution comprising the magnetic core to the solution comprising the outer shell.

In a particular embodiment of the method to carry out step c) of the method II of the invention of the invention, step ii) is done is a vacuum.

In a particular embodiment of the method to carry out step c) of the method II of the invention of the invention, step iii) is done by ultrasonic bath.

In a particular embodiment of the method to carry out step c) of the method II of the invention, further comprising a step of cleaning the redispersed solid by centrifuging following by dispersing the pellet in a buffer.

The term "buffer" as used herein refers to one or more compositions, or aqueous solutions thereof, that resist fluctuation in the pH when an acid or an alkali is added to the solution or composition that includes the buffer. This resistance to pH change is due to the buffering properties of such solutions, and may be a function of one or more specific compounds included in the composition. Thus, solutions or other compositions exhibiting buffering activity are referred to as buffers or buffer solutions. Buffers generally do not have an unlimited ability to maintain the pH of a solution or composition; rather, they are typically able to maintain the pH within certain ranges, for example from a pH of about 5 to 7. Examples of buffers, without limitation, include PBS, MES, bis-Tris, ADA, ACEs, PIPES, MOPSO, Propano bis-Tris, BES, TES, HEPES, DIPSO, MOBS, TAPSO, Tris, HEPPSO, POPSO, TEA, EPPS, Tricina, Gly-Gly, Bicina, HEPBS, TAPS, AMPD, CHES, CAPSO, AMP, CAPS, CABS, SBBS, preferably SBBS or PBS.

### Fourth step of method II

Step d) of the method II of the invention refers to the functionalization of the nanoparticles obtained in step c) with avidin protein. In a particular embodiment of method II of the invention, step d) is carried out by contacting activated avidin protein to the nanoparticles obtained in step c), wherein said activated avidin protein contains a reactive group capable of reacting with the thiol group present in the PMAO-PEG copolymer, wherein said contacting is carried out under conditions adequate for the formation of a bond between the reactive group in the avidin protein and the thiol group in the PMAO-PEG copolymer.

In a particular embodiment of method II of the invention, the avidin protein is deglycosylated.

"Activated", as used herein when referring to a molecule, refers to a modified version of the molecule which contains a chemical modification whereby said molecule is capable to chemically react in a manner not previously present in the molecule (for instance, the activation adds a moiety not present previously, allowing for a bond that was unfeasible before) or with an increased reactivity (meaning that the reaction of the molecule with another molecule requires a lower activation energy than in the inactivated state). The present invention contemplates the possibility of activating the one or two of the members of the binding pair and/or the long noncoding RNA and/or the amphiphilic polymer, and then contacting the activated one or two of the members of the binding pair and/or the long noncoding RNA and/or the amphiphilic polymer with the activated one or two of the members of the binding pair and/or the long noncoding RNA and/or the amphiphilic polymer. In both cases, the activation of the molecule is usually carried out by reacting the molecule to be activated with a reagent that introduces the reactive group in a suitable moiety in the molecule to be activated. Examples of reactive groups that allow the one or two of the members of the binding pair and/or the long noncoding RNA and/or the amphiphilic polymer to be activated include, but are not limited to: carboxyl, amine, imine, thiol, sulfone, hydroxyl, sulfate, and phosphate moieties, among many others which are commonly known to the person skilled in the art. The activated form of the one or two of the members of the binding pair and/or the long noncoding RNA and/or the amphiphilic polymer is also herein referred to as the "activated member of the member of the binding pair" or "activated long noncoding RNA" or "activated amphiphilic polymer".

In a particular embodiment of method II of the invention, the active group is maleimide. In another particular embodiment of the method II of the invention, the active group is a handle of a click chemistry pair of handles, as previously described.

In a particular embodiment of method II of the invention, the long noncoding RNA is WAKMAR2, preferably the long noncoding RNA comprises a nucleotide sequence with at least 90% identity to SEQ ID NO: 1 or a functionally equivalent thereof.

### Fifth step of method II

The final step of method II refers to the binding of the long noncoding RNA to the outer shell of the nanoparticles obtaining the nanoparticles of the invention. In a particular embodiment of method II, step e) is carried out by mixing the functionalized PAMO-PEG obtained in step d) with the nanoparticles obtained in step c) in RNase free water at a ratio of 10 long noncoding RNA to one nanoparticle.

In a particular embodiment of method II of the invention, step e) is carried out with a ratio of avidin to nanoparticles (NA-to-NP) higher than the ratio of long noncoding RNA to nanoparticles (IncRNA-to-NP). In another particular embodiment of method II of the invention, step e) is carried out with a ratio of avidin to nanoparticles (NA-to-NP) lower than the ratio of long noncoding RNA to nanoparticles (IncRNA-to-NP).

### Uses of the invention

The nanoparticles of the invention find uses as vehicles of delivery of long noncoding RNAs to different tissues and locations.

A further aspect of the present invention refers to the nanoparticles of the invention for use in medicine.

A further aspect of the present invention refers to the nanoparticles of the invention for use in the treatment of a disease which is treatable by the expression of the long noncoding RNA present in the nanoparticle, from here onwards the use I of the invention.

As used herein, the terms "treat", "treatment", "treatment", or "amelioration" refer to therapeutic treatment, the purpose of which is to reverse, reduce, suppress, delay or stop the progression, symptoms or severity of the condition associated with the disease or disorder. The term "treatment" includes reducing or alleviating at least one adverse effect or condition of a condition, such as chronic inflammation, cardiac disease or cancer, a disease or disorder. Treatment is usually "effective" when one or more symptoms or clinical markers are reduced. Alternatively, treatment is "effective" if disease progression is delayed or halted. That is, "treatment" includes not only the improvement of symptoms or markers, but also the interruption of at least a condition that indicates the progression or worsening of symptoms that would be expected in the absence of treatment. The beneficial or desirable clinical outcome, whether detectable or not, is a reduction in one or more symptoms, a reduction in the extent of the disease, a stable (ie, not aggravated) condition of the disease, a disease These include, but are not limited to, delayed or slowed progression, amelioration or alleviation of the disease state, and remission (partial or total). The term "treatment" of a disease also includes providing relief from symptoms or side effects of the disease (including symptomatic treatment). In some embodiments, treating inflammation includes reducing or suppressing inflammation markers. In some embodiments treating cardiac disease includes reducing or managing abnormal heart rhythms, reducing hypertension, reducing cholesterol, reducing blood coagulation, increasing diuresis, relaxing blood vessels, reducing platelet aggregation, releasing obstructed blood vessels. in some embodiments cancer includes reducing tumor volume, reducing the number of cancer cells, suppressing cancer metastasis, prolonging life, reducing cancer cell growth, reducing cell survival, or reducing cancerous status It involves amelioration of the various physiological symptoms involved.

In a particular embodiment of the use I of the invention the treatable disease is selected from a group consisting of: cardiovascular disease, inflammatory and autoimmune disorders, neurodegenerative diseases, sepsis, chronic wounds and cancer.

In a particular embodiment of the use I of the invention the treatable disease is selected from a group consisting of: parkinson's disease (PD), diabetic retinopathy, and osteoarthritis.

As used herein, the term "cardiovascular disease" is intended to refer to all pathological states leading to a narrowing and/or occlusion of blood vessels throughout the body. In particular, the term "cardiovascular disease" refers to conditions including atherosclerosis, thrombosis, and other related pathological states, especially within arteries of the heart and brain. In a particular embodiment of the use I of the invention the cardiovascular disease is selected from a group consisting of: atherosclerosis, thrombosis, coronary artery disease, arrhythmia, congenital heart disease, hypertension, peripheral heart disease, cardiomyopathy, angina, pericarditis, rheumatic heart disease, aortic disease, cerebrovascular disease, and abnormal heart rhythms, preferably atherosclerosis.

The term "inflammation" as used herein refers to local response to noxious endogenous or exogenous stimuli causing tissue injury, characterized by capillary dilation and leukocyte infiltration and the typical signs and symptoms of inflammation, which include swelling, redness, increased local and/or general temperature and pain. In a particular embodiment of the use I of the invention, the inflammatory disease is chronic inflammation, rheumatoid arthritis, arthritis, respiratory inflammatory diseases, such as, asthma, chronic obstructive pulmonary disease (COPD), and pulmonary fibrosis.

The term "Parkinson's disease" as used herein refers to a disease or syndrome characterized by progressive degeneration of nigrostriatal dopamine neurons and the appearance of Lewy bodies and/or Lewy neurites appearing in various brain regions.

The term "diabetic retinopathy" includes retinopathy (i.e., a disease of the retina) caused by complications of diabetes, which can eventually lead to blindness. Diabetic retinopathy may cause no symptoms, mild vision problems, or even blindness. Diabetic retinopathy is the result of microvascular retinal changes.

The term "osteoarthritis" as used herein means the most common form of arthritis. The term "osteoarthritis" includes primary osteoarthritis and secondary osteoarthritis. Osteoarthritis can be caused by the breakdown of cartilage. A small amount of cartilage may fall off, causing pain and swelling in the joint between the bones. Over time, this cartilage can wear down completely, causing bone to rub against bone. Osteoarthritis can affect any joint, but usually involves the hands, shoulders, and weight-bearing joints such as the hips, knees, feet, and spine.

The term "sepsis" as used herein refers to a serious condition resulting from the presence of harmful microorganisms in the blood or other tissues and the body's response to their presence, potentially leading to the malfunctioning of various organs, shock, and death.

The term "chronic wounds" refers to wounds that show no tendency to heal after 6-8 weeks.

The term "cancer" or "tumor" or "tumor disease", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis. Cancers usually share some of the following characteristics: sustaining proliferative signaling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumor cells resemble, which is therefore presumed to be the origin of the tumor.

Examples of cancer or tumor include without limitation, breast, heart, lung, small intestine, colon, spleen, kidney, bladder, head, neck, ovarian, prostate, brain, rectum, pancreas, skin, bone, bone marrow, blood, thymus, uterus, testicles, hepatobiliary and liver tumors. In particular, the tumor/cancer can be selected from the group of adenoma, angiosarcoma, astrocytoma, epithelial carcinoma, germinoma, glioblastoma, glioma, hemangioendothelioma, hepatoblastoma, leukaemia, lymphoma, medulloblastoma, melanoma, neuroblastoma, hepatobiliary cancer, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytictumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelialtumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, vipoma, Wilm's tumor.

In a particular embodiment of the use I of the invention, the cancer is a solid tumor. In another particular embodiment of the use I of the invention, the cancer is a metastatic cancer.

In another particular embodiment of use I of the invention, the long noncoding RNA present in the nanoparticles is one selected from column 1 of Table 1 and the treatable disease is the corresponding disease from column 2 of Table 1 to the long noncoding RNA selected.

Another aspect of the present invention relates to the nanoparticle of the invention for use in the treatment of chronic inflammation, chronic wounds and/or cancer, wherein the nanoparticles long non coding RNA is WAKMAR2, preferably the long noncoding RNA comprises a nucleotide sequence with at least 90% identity to SEQ ID NO: 1 or a functionally equivalent thereof, preferably wherein the cancer is a solid tumor or a metastatic cancer.

The following invention is further defined by the following aspects:
1. A nanoparticle comprising a magnetic core and an outer shell wherein the outer shell comprises a long noncoding RNA.
2. The nanoparticle according to aspect 1 wherein the magnetic core is made of an iron oxide.
3. The nanoparticle according to aspect 2 wherein the iron oxide is selected from Fe₂O₃, and Fe₃O₄.
4. The nanoparticle according to aspect 3 wherein the iron oxide is Fe₃O₄.
5. The nanoparticle according to any one of aspects 1 to 4 wherein the outer shell contains an amphiphilic polymer.
6. The nanoparticle according to aspect 5 wherein the amphiphilic polymer is selected from a group consisting of: poly(ethylene glycol), poly(styrene-co-vinylpyrrolidone), poly(lauryl methacrylate), poly(oligo ethylene glycol methacrylate), poly(acrylic acid), poly(maleic anhydride-alt-1-octadecene), and any combination thereof.
7. The nanoparticle according to aspect 6 wherein the amphiphilic polymer is a copolymer, preferably the copolymer poly(maleic anhydride-alt-1-octadecene)-poly(ethylene glycol) (PMAO-PEG).
8. The nanoparticle according to any of aspects 5 to 7 wherein the amphiphilic polymer is functionalized with a first member of a binding pair of molecules.
9. The nanoparticle according to aspect 8 wherein each nanoparticle comprises between 1 to about 10 copies of the first member of the binding pair of molecules.
10. The nanoparticle according to aspect 9 wherein each nanoparticle comprises about 3 to about 5 copies of the first member of the binding pair of molecules.
11. The nanoparticle according to any of aspects 8 to 10 wherein the first member of the binding pair of molecules is an avidin protein.
12. The nanoparticle according to aspect 11 wherein the avidin protein is selected form a group consisting of: streptococcus avidin (streptavidin), chicken avidin (extravidin), neutralite avidin (neutravidin).
13. The nanoparticle according to aspect 11 or 12 wherein the avidin protein is deglycosylated.
14. The nanoparticle according to any one of aspects 8 to 13 wherein the long noncoding RNA is functionalized with a second member of the binding pair of molecules which is bound to the first member of the binding pair of molecules.
15. The nanoparticle according to aspect 14 wherein the second member of the binding pair of molecules is biotin and the first member of the binding pair is avidin according to any of aspects 11 to 13.
16. The nanoparticle according to aspect 1 wherein the magnetic core is Fe₃O₄, the outer shell contains a PMAO-PEG copolymer, wherein the molecules of PMAO-PEG copolymer are functionalized with avidin protein and wherein the long noncoding RNA is functionalized with biotin and the biotin is bound to the avidin protein.
17. The nanoparticle according to any one of aspects 1 to 16 wherein the long noncoding RNA is the product resulting from the transcription of the gene WAKMAR2 (NCBI Reference Sequence: NR_049793.1, Mar25, 2023).
18. The nanoparticle according to aspect 17 wherein the long noncoding RNA has a nucleotide sequence comprising at least 90% identity to SEQ ID NO: 1 or a functionally equivalent thereof.
19. The nanoparticles according to any one of aspects 1 to 18 further comprising a marker bound to the outer shell.
20. The nanoparticle according to any one of aspects 1 to 19 wherein the nanoparticles have an average size of between about 15 nm and 25 nm.
21. The nanoparticle according to aspect 20 wherein the nanoparticles have an average size of about 18 nm to about 22 nm.
22. The nanoparticles according to any one of aspects 1 to 21 wherein the nanoparticles have a saturation magnetization at room temperature of more than 90 A·m²/kg.
23. A method for obtaining a nanoparticle according to any of aspects 1 to 22 comprising the steps of:
   a) providing a nanoparticle comprising a magnetic core and an outer shell wherein the outer shell is functionalized with a first member of a binding pair of molecules;
   b) providing an long noncoding RNA functionalized with a second member of the binding pair of molecules;
   c) bringing the nanoparticle of step a) into contact with the long noncoding RNA of step b) in conditions such that the second member of the binding pair of molecules binds to the first member of a binding pair of molecules.
24. The method according to aspect 23 wherein the magnetic core is made of iron oxide.
25. The method according to aspect 24 wherein the iron oxide is selected from FeO, Fe₂O₃, and Fe₃O₄.
26. The method according to aspect 25 wherein the iron oxide is Fe₃O₄.
27. The method according to any one of aspects 23 to 26 wherein the outer shell contains an amphiphilic polymer.
28. The method according to aspect 27 wherein the amphiphilic polymer is selected from a group consisting of: poly (ethylene glycol), poly(styrene-co-vinylpyrrolidone), poly(lauryl methacrylate), poly(oligo ethylene glycol methacrylate), poly(acrylic acid), poly(maleic anhydride-alt-1-octadecene), and any combination thereof.
29. The method according to aspect 28 wherein the amphiphilic polymer is a copolymer, preferably the copolymer is poly(maleic anhydride-alt-1-octadecene)-poly(ethylene glycol) (PMAO-PEG).
30. The method according to any of aspects 23 to 29 wherein the first member of the binding pair of molecules is an avidin protein.
31. The method according to aspect 30 wherein the avidin protein is selected form a group consisting of: streptococcus avidin (streptavidin), chicken avidin (extravidin), neutralite avidin (neutravidin),
32. The method according to aspect 30 or 31 wherein the avidin protein is deglycosylated.
33. The method according to any of aspects 23 to 32 wherein the second member of the binding molecules is biotin.
34. The method according to any of aspects 30 to 33 wherein the long noncoding RNA binds to the outer shell through a non-covalent bond between the avidin and the biotin.
35. The method according to any of aspects 23 to 34 wherein the long noncoding RNA is the product resulting from the transcription of the gene WAKMAR2 (NCBI Reference Sequence: NR_049793.1, Mar25, 2023).
36. The method according to aspect 35 wherein the long noncoding RNA has a nucleotide sequence comprising at least 90% identity to SEQ ID NO: 1 or a functionally equivalent thereof.
37. A method for obtaining a nanoparticle according to any of aspects 1 to 22 comprising the steps of:
   a) providing a suspension comprising a magnetic nanoparticle;
   b) providing a solution comprising an amphiphilic polymer, wherein the polymer is optionally modified with a first member of a binding pair;
   c) mixing the suspension of step a) and the solution of step b) such that the amphiphilic polymer binds to and coats the magnetic nanoparticle, obtaining a polymer-coated nanoparticle;
   d) if the amphiphilic polymer used in step b) is not modified with a first member of a binding pair in step b), then functionalizing the amphiphilic polymer of the polymer-coated nanoparticles obtained in step c) with a first member of a binding pair;
   e) bringing into contact the polymer-coated nanoparticles obtained in step d) with a long noncoding RNA functionalized with a second member of the binding pair, under conditions adequate for the binding of the first and second members of the binding pair.
38. The method according to aspect 37 wherein the magnetic nanoparticle is made of iron oxide.
39. The method according to aspect 38 wherein the iron oxide is selected from Fe₂O₃, and Fe₃O₄.
40. The method according to aspect 39 wherein the iron oxide is Fe₃O₄.
41. The method according to any of aspects 37 to 40 wherein the suspension comprising the magnetic nanoparticle of step a) is obtained by a method comprising the following steps:
   i) dissolving iron (III) oleate (FeOl) in a mixture of 1-octadecene and dibenzyl ether with oleic acid obtaining a mixture;
   ii) heating the mixture of step i) at 10°C/min from room temperature up to 190°C;
   iii) heating the heated mixture of step ii) at 3°C/min from 190°C to 330 °C, allowing reflux for at least 60 min;
   iv) allow the mixture of step iii) to cool to room temperature, obtaining a solution with the magnetic core,
   wherein all steps i)-iv) are carried out under mechanical stirring.
42. The method according to aspect 41 wherein the concentration of FeOl in step i) is 0.25 mmol/mL, the mixture of 1-octadecene and dibenzyl ether is at a ratio of 1:2 of 1-octadecene to dibenzyl ether and the concentration of oleic acid is of 0.5 mmol/mL.
43. The method according to aspect 41 or 42 wherein the mechanical stirring is done by rotation at 120 rpm.
44. The method according to any one of aspects 41 to 43 wherein the mixture obtained in step iv) is further cleaned by centrifugation using tetrahydrofuran and ethanol at a temperature of less or equal to 6 °C.
45. The method according to any of aspects 37 to 44 wherein the amphiphilic polymer of step b) is selected from a group consisting of: poly (ethylene glycol), poly(styrene-co-vinylpyrrolidone), poly(lauryl methacrylate), poly(oligo ethylene glycol methacrylate), poly(acrylic acid), poly(maleic anhydride-alt-1-octadecene), and any combination thereof.
46. The method according to aspect 45 wherein the amphiphilic polymer is a copolymer, preferably the copolymer is poly(maleic anhydride-alt-1-octadecene)-poly(ethylene glycol) (PMAO-PEG).
47. The method according to aspect 46 wherein the amphiphilic polymer is the copolymer poly(maleic anhydride-alt-1-octadecene)-poly(ethylene glycol) (PMAO-PEG) and wherein the solution comprising the PMAO-PEG copolymer of step b) is obtained by a method comprising the steps of:
   i) adding amine-poly(ethylene glycol)-thiol to a solution of poly(maleic anhydride-alt-1-octadecene) in an immiscible solvent and stirring the obtained mixture at room temperature overnight;
   ii) adding poly(ethylene glycol)-amine to the mixture obtained in ii) and stirring the obtained mixture at room temperature for at least 12h, preferably at least 24h, obtaining the PMAO-PEG copolymer.
48. The method according to aspect 47 wherein the solution of poly(maleic anhydride-alt-1-octadecene) in step i) is at a concentration of 10 mg/ml.
49. The method according to aspect 47 or 48 wherein amine-poly(ethylene glycol)-thiol is added in step i) at a molar ratio of between 5-75% of amine-poly(ethylene glycol)-thiol to poly(maleic anhydride-alt-1-octadecene).
50. The method according to any one of aspects 47 to 49 wherein the method further comprises an intermediate step after step i) and before step ii) of adding 5-TAMRA cadaverine to the mixture obtained in step i) and stirring the obtained mixture at room temperature overnight.
51. The method according to aspect 50 wherein the 5-TAMRA cadaverine is added at a concentration of between5-35% w/v.
52. The method according to any one of aspects 47 to 51 wherein the poly(ethylene glycol)-amine is added in step ii) at a concentration of 75% w/v.
53. The method according to any one of aspects 47 to 52 wherein the immiscible solvent is chloroform.
54. The method according any one of aspects 37 to 53 wherein step c) is carried out by a method comprising the following steps:
   i) stepwise adding the suspension comprising the magnetic nanoparticle to the solution comprising the amphiphilic polymer until the suspension and solution are fully mixed;
   ii) evaporating all the solvent of the mixture obtained in step ii) obtaining a solid;
   iii) redispersing the solid obtained in step iii) in a buffer solution, obtaining a polymer-coated nanoparticle.
55. The method according to aspect 54 wherein the stepwise addition is done by adding a drop at a time of the solution comprising the magnetic core to the solution comprising the amphiphilic polymer.
56. The method according to aspect 54 or 55 wherein step ii) is done in a vacuum.
57. The method according to any of aspects 54 to 56 wherein step iii) is done by ultrasonic bath.
58. The method according to any of aspects 54 to 57 further comprising a step of cleaning the redispersed solid by centrifuging following by dispersing the pellet in a buffer.
59. The method according to any one of aspects 37 to 58 wherein step d) is carried out by contacting the activated first member of the binding pair to the nanoparticles obtained in step c), wherein said activated first member of the binding pair contains a reactive group capable of reacting with a receptive group present in the amphiphilic polymer, wherein said contacting is carried out under conditions adequate for the formation of a bond between the reactive group in the first member of the binding pair and the receptive group in the amphiphilic polymer.
60. The method according to aspect 59 wherein the first member of the binding pair of molecules is an avidin protein.
61. The method according to aspect 60 wherein the avidin protein is selected form a group consisting of: streptococcus avidin (streptavidin), chicken avidin (extravidin), neutralite avidin (neutravidin),
62. The method according to aspect 60 or 61 wherein the avidin protein is deglycosylated.
63. The method according to any of aspects 59 to 62 wherein the reactive group is maleimide and the receptive group is thiol.
64. The method according to any of aspects 59 to 63 wherein the second member of the binding molecules is biotin.
65. The method according to any one of aspects 37 to 64 wherein the long noncoding RNA is WAKMAR2, preferably the long noncoding RNA comprises a nucleotide sequence with at least 90% identity to SEQ ID NO: 1 or a functionally equivalent thereof.
66. The nanoparticles according to any one of aspects 1 to 22 for use in medicine.
67. The nanoparticles according to any one of aspects 1 to 22 for use in the treatment of a disease which is treatable by expression of the long noncoding RNA present in the nanoparticles.
68. The nanoparticles according to aspect 17 or 18 for the use in the treatment of chronic inflammation, chronic wounds and/or cancer.
69. The nanoparticles for use according to aspect 68 wherein the cancer is a solid tumor.
70. The nanoparticles for use according to aspect 68 or 69 wherein the cancer is a metastatic cancer.
71. The nanoparticles for use according to any one of aspects 68 to 70 wherein the cancer is gastric cancer or colon cancer.
72. The nanoparticles for use according to any one of aspects 68 to 71 wherein the long noncoding RNA is WAKMAR2 and the cancer cells are characterized by low expression of WAKMAR2.
73. The nanoparticles for use according to aspect 68 wherein the chronic inflammation is intestinal chronic inflammation, preferably colon chronic inflammation.
74. A method to increase endogenous levels of a long noncoding RNA comprising the step of transfecting cells with the nanoparticles according to any of aspects 1 to 22.
75. The method according to aspect 74 wherein the long noncoding RNA is WAKMAR2 and the cells are characterized by having low expression of WAKMAR2.

The invention will be described by way of the following examples which are to be considered as merely illustrative and not limitative of the scope of the invention.

### EXAMPLES

### Materials and Methods

Sodium oleate (97%) was purchased from TCI America, poly (ethylene glycol)-amine (PEG-NH₂) and amine-poly (ethylene glycol)-thiol (NH₂-PEG-SH) from LaysanBio, ethanol from Panreac (96%), phosphate-buffered saline (PBS) from Gibco and maleimida-activated NeutrAvidin (NA) from Thermo Fischer Scientific. Iron (III) chloride hexahydrate (99%) and other solvents and reagents were purchased from Merck and used without further purification: 5-TAMRA cadaverine, oleic acid (90%), 1-octadecene (ODE) (90%), dibenzyl ether (DBE) (98%), chloroform (>99%), tetrahydrofuran (>99.9%), hexane (99%), poly(maleic anhydride-alt-1-octadecene) (PMAO) (MW = 30,000-50,000 Da), sodium hydroxide (>99.9%) and boric acid (99.9%).

### Synthesis of Iron (III) Oleate

For synthetizing iron (III) oleate (FeOl), 40 mmol of FeCl₃·6H₂O and 124 mmol of sodium oleate are added to a solvent mixture consisting of 140 mL hexane, 80 mL ethanol, and 60 mL D.I. H₂O. The mixture is heated to reflux (60 °C) for one hour under N₂ atmosphere. After cooling it down to room temperature, the aqueous phase is removed using a separatory funnel and the organic phase containing the iron oleate complex is washed with D.I H₂O (100 mL x 3). Finally, the organic phase with the FeOl is heated at 110 °C overnight, to remove all the remnant solvents. The resulting product, once cooled to room temperature, is a black-brownish viscous solid.

### Synthesis of Fe₃O₄ NPs

FeOl (5 mmol) is dissolved in a mixture of 14 mL 1-octadecene (ODE) and 7 mL dibenzyl ether (DBE), with oleic acid (10 mmol). The mixture is heated in two steps under N₂ atmosphere, using a temperature controller, first at 10 °C/min from RT to 190 °C and second, at 3 °C/min from 190 °C to 330 °C. Reflux is maintained for 60 minutes, and then it is allowed to cool to RT. The entire synthesis is carried out under mechanical stirring at 120 rpm. The final product is cleaned by centrifugation using 10 mL THF and 20 mL EtOH (20.000 RPM x 3) at 6 °C. The cleaned MNPs are redispersed into 15 mL CHCl₃ and stored in the fridge at 6 °C.

### Synthesis of the thiolated-PMAO-PEG Copolymer and Coating Procedure of MNPs

The synthesis of the thiolated and fluorescent PMAO-PEG copolymer takes three steps. First, NH₂-PEG-SH, is added to a solution of 10 mg/mL of poly (maleic anhydride-alt-1-octadecene) (PMAO) in CHCl₃, at NH₂-PEG-SH /PMAO monomer molar ratio of 10%, adding enough CHCl₃ to reach a volume of 500 µL. Then, the solution is stirred at RT overnight. Second, 10% of 5-TAMRA cadaverine is added to the mixture and again, the solution is stirred at RT overnight. Finally, 75% of 20.000 Da PEG-NH₂ is added, and the solution is stirred for a day.

To perform the functionalization of NPs with the copolymer, the necessary volume of copolymer solution (50 PMAO monomers per nm² of NP) is placed in a microcentrifuge tube. Then Fe₃O₄ NP colloid is added to the PMAO-PEG dropwise. After addition, the tubes are immersed in an ultrasonic bath for 30 minutes. The mixture is then transferred to a 100 mL round flask. Next, all the solvent is evaporated under vacuum and the solid adhered to the glass is redispersed in 2 mL of CHCl₃, using the ultrasonic bath. The solvent is evaporated again in vacuum and the solid is redispersed in 1 mL of SBBS buffer solution (sodium borate-buffered saline, pH = 9.45) using ultrasound. Next, the dispersion is placed in a microcentrifuge tube, centrifuged at 14.000 RPM for 30 min and dispersed in 500 µL PBS (phosphate buffered saline), prepared in RNase free water. The cleaning is repeated as many times as necessary, until the supernatant is colorless.

### Coating of Thiol-labelled NPs with Maleimide-activated Neutravidin

For the conjugation of thiol-coated NPs, 50 maleimide-activated NA molecules per Fe₃O₄ NP are used. The thiol-labelled NPs dispersed in RNase free PBS are added drop-by-drop (10 µL) on top of the maleimide-activated neutravidin, dissolved in RNase free PBS at 2 mg/mL, the mixture is gently stirred overnight. Then, the Fe₃O₄@NA NPs are cleaned through centrifugation at 13.000 RPM, 6 °C for 30 minutes three times.

### Coating of Neutravidin-Labelled NPs with IncRNA-biotin

The Fe₃O₄@NA NPs are mixed with *WAKMAR2* IncRNA dissolved in RNase free water, at 10 IncRNA/NP ratio. The volume is brought to 200 µL and the mixture is stirred during 1 hour at RT. Free IncRNA molecules are removed by magnetic separation. Fe₃O₄@IncRNA NPs are redispersed in RNase free PBS.

### In vitro transcription of biotinylated RNA

*WAKMAR2* was amplified by PCR from an overexpression plasmid using a Forward T7 promoter primer and a reverse primer specific to the IncRNA 3' end. The PCR product was purified using a column-based kit (Machinery Nagel) and used for in vitro transcribing biotinylated RNA using the T7 polymerase (Takara) and RNA biotin labeling kit (Roche).

### Results

Our non-viral RNA delivery system has been produced in three steps: First, magnetite NPs have been functionalized with protein molecules of NeutrAvidin (Fe₃O₄@NA). Secondly, biotinylated-IncRNA (*WAKMAR2*) has been in vitro transcribed. Finally, the conjugation between Fe₃O₄@NA and biotinylated-IncRNA has been carried out to produce a IncRNA delivery nanoplatform with magnetic targeting potential.

Magnetite (Fe₃O₄) NPs of great quality (highly homogeneous in size, shape and composition with high saturation magnetization at RT (> 90 A·m²/kg), see **Figure 1****)** have been synthesized by adapting previously optimized chemical method (Chem. Mater. 2021, 33, 8693). These cuboctahedral NPs of 20 nm have been coated with a fluorescent copolymer (composed of PMAO, thiolated-PEG and rhodamine) by slightly modifying recently refined process (ACS Appl. Mater. Interfaces 2022, 14, 50033). The high molecular weight of the copolymer and the optimized coating protocol (described above) result in magnetic nanoformulations with minimal aggregation (see D_{H} values in **Table 1).** Then, maleimide-activated NeutrAvidin (NA) molecules have been attached to the polymeric coating of the nanoparticles by thiol-maleimide "click chemistry". We have successfully introduced variable amounts of NA on the surface, ranging from 3 to 20 NA per NP. As shown in **Figure 2** and **Table 2,** increasing the NA content leads to thicker organic coatings and larger hydrodynamic diameters.

For the in vitro transcription of the biotinylated-IncRNA *WAKMAR2,* IncRNA has been amplified by PCR with the addition of a T7 promoter in its 5' side. After PCR purification, the DNA template has been used for in vitro transcription using a T7 polymerase and biotinilated oligos. After transcription, the RNA has been purified and treated with DNAse I.

The Fe₃O₄@WAKMAR2 nanoplatforms (see **Figure 3a****)** have been obtained by mixing and stirring Fe₃O₄@NA and WAKMAR2-biotin solutions at RT for one hour. Thanks to the strong NeutrAvidin-biotin bind an efficient conjugation has been achieved. One of the major advantages of our protocol is that the amount of IncRNA incorporated into the nanosystem can be precisely controlled. We have recently confirmed that this amount can be tuned over a relatively wide range, specifically between 2 and 40 IncRNA molecules per nanoparticle (see **Figure 3b** and **3c****).** Two strategies have been identified to modulate the final amount of IncRNA in the nanovehicle: (i) increasing the amount of NA while maintaining a constant initial IncRNA-to-NP ratio (Figure 2b), or (ii) keeping the NA-to-NP ratio constant while increasing the initial IncRNA-to-NP ratio (Figure 2c). Additionally, it has been demonstrated that conjugating a different type of IncRNA onto the surface of the nanosystem is entirely feasible. As shown in Figure 3d, when the standard conjugation protocol is applied to LINC00467 (using Fe₃O₄@NA₇ and an initial IncRNA/NP ratio of 10:1), the resulting final ratio of IncRNA per nanoparticle is approximately 4:1.

Studies performed by our laboratories have confirmed the ability of the IncRNA loaded MNPs (Fe₃O₄@*WAKMAR2*) to enter the cells and increase the endogenous IncRNA levels **(****Figure 4** **a,b).** Interestingly, the incorporation of this complex into the intestinal epithelial cells does not induce an inflammatory response in these cells **(****Figure 4c****).** Lastly, RNA stability studies have shown that the IncRNAs bound to the MNPs have a significantly lower degradation rate in both FBS and human serum **(****Figure 4d** and data not shown). Moreover, complexes stored for up to 1 month are still able to increase endogenous IncRNA levels in cells **(****Figure 4e****).** All together, these data stress the feasibility of this approach for the safe and efficient delivery of anti-inflammatory IncRNAs into intestinal cells.

**Table 2. Polydispersity index (PDI), average hydrodynamic diameter (given as number, D_{H}), and zeta potential (PZ) for Fe₃O₄@OA in CHCl₃, and for Fe₃O₄@PEG-SH and Fe₃O₄@NA in distilled water and physiological medium (PBS)**

| **Sample** | **Dispersante** | **PDI** | **D_{H} (nm)** | **P_{Z} (mV)** |
|---|---|---|---|---|
| Fe₃O₄@AO | CHCl₃ | 0.112 | 77 (15) | - |
| Fe₃O₄@PEG-SH | H₂O | 0.145 | 71 (12) | -20 (3) |
| | PBS | 0.272 | 78 (7) | |
| Fe₃O₄@NA₃ | H₂O | 0.380 | 83 (16) | -18 (5) |
| | PBS | 0.327 | 65 (11) | |
| Fe₃O₄@NA₇ | H₂O | 0.397 | 91 (29) | -22 (3) |
| | PBS | 0.487 | 70 (6) | |
| Fe₃O₄@NA₂₀ | H₂O | 0.787 | 378 (102) | -21 (4) |
| | PBS | 0.858 | 279 (35) | |

## Claims

1. A nanoparticle comprising a magnetic core and an outer shell wherein the outer shell comprises a long noncoding RNA.

2. The nanoparticle according to claim 1 wherein the magnetic core is made of an iron oxide.

3. The nanoparticle according to claim 2 wherein the iron oxide is Fe₃O₄.

4. The nanoparticle according to any one of claims 1 to 3 wherein the outer shell contains an amphiphilic polymer.

5. The nanoparticle according to claim 4 wherein the amphiphilic polymer is a copolymer, preferably the copolymer poly(maleic anhydride-alt-1-octadecene)-poly(ethylene glycol) (PMAO-PEG).

6. The nanoparticle according to claim 1 wherein the magnetic core is Fe₃O₄, the outer shell contains a PMAO-PEG copolymer, wherein the molecules of PMAO-PEG copolymer are functionalized with avidin protein and wherein the long noncoding RNA is functionalized with biotin and the biotin is bound to the avidin protein.

7. The nanoparticle according to any one of claims 1 to 6 wherein the long noncoding RNA has a nucleotide sequence comprising at least 90% identity to SEQ ID NO: 1 or a functionally equivalent thereof.

8. A method for obtaining a nanoparticle according to any of claims 1 to 7 comprising the steps of:
a) providing a nanoparticle comprising a magnetic core and an outer shell wherein the outer shell is functionalized with a first member of a binding pair of molecules;
b) providing a long noncoding RNA functionalized with a second member of the binding pair of molecules;
c) bringing the nanoparticle of step a) into contact with the long noncoding RNA of step b) in conditions such that the second member of the binding pair of molecules binds to the first member of a binding pair of molecules.

9. A method for obtaining a nanoparticle according to any of claims 1 to 7 comprising the steps of:
a) providing a suspension comprising a magnetic nanoparticle;
b) providing a solution comprising an amphiphilic polymer, wherein the polymer is optionally modified with a first member of a binding pair;
c) mixing the suspension of step a) and the solution of step b) such that the amphiphilic polymer binds to and coats the magnetic nanoparticle, obtaining a polymer-coated nanoparticle;
d) if the amphiphilic polymer used in step b) is not modified with a first member of a binding pair in step b), then functionalizing the amphiphilic polymer of the polymer-coated nanoparticles obtained in step c) with a first member of a binding pair;
e) bringing into contact the polymer-coated nanoparticles obtained in step d) with a long noncoding RNA functionalized with a second member of the binding pair, under conditions adequate for the binding of the first and second members of the binding pair.

10. The nanoparticles according to any one of claims 1 to 7 for use in medicine.

11. The nanoparticles according to any one of claims 1 to 7 for use in the treatment of a disease which is treatable by expression of the long noncoding RNA present in the nanoparticles.

12. The nanoparticles according to claim 7 for the use in the treatment of chronic inflammation, chronic wounds and/or cancer.

13. The nanoparticles for use according to claim 12 wherein the cancer is gastric cancer or colon cancer.

14. The nanoparticles for use according to claim 12 or 13 wherein the long noncoding RNA is WAKMAR2 and the cancer cells are **characterized by** low expression of WAKMAR2.

15. The nanoparticles for use according to claim 14 wherein the chronic inflammation is intestinal chronic inflammation, preferably colon chronic inflammation.
